(19) Europäisches Patentamt
European Patent Office
Office européen des brevets

(11) EP 0 612 730 B1

(12) **EUROPEAN PATENT SPECIFICATION**

(45) Date of publication and mention of the grant of the patent:
**02.07.1997 Bulletin 1997/27**

(51) Int. Cl.[6]: **C07D 221/28**, A61K 31/485, C07D 401/12, C07D 409/12, C07D 401/14, C07D 417/12

(21) Application number: **93120498.6**

(22) Date of filing: **20.12.1993**

(54) **O-aryl ethers of morphinans**
Morphinan-O-Arylether
O-Aryléthers de morphinane

(84) Designated Contracting States:
**AT BE CH DE DK ES FR GB GR IE IT LI LU MC NL PT SE**

(30) Priority: **06.01.1993 US 1135**

(43) Date of publication of application:
**31.08.1994 Bulletin 1994/35**

(73) Proprietor: **F. HOFFMANN-LA ROCHE AG**
**4002 Basel (CH)**

(72) Inventors:
- **Mohacsi, Erno**
**Summit, New Jersey 07901 (US)**
- **O'Brien, Jay Philip**
**Cedar Grove, New Jersey 07009 (US)**

(74) Representative: **Mahé, Jean et al**
**F.Hoffmann-La Roche AG**
**Patent Department (PLP),**
**124 Grenzacherstrasse**
**4070 Basel (CH)**

(56) References cited:
**EP-A- 0 270 290** **DE-A- 2 754 062**
**US-A- 4 113 729** **US-A- 4 163 853**

- **J. MED. CHEM. vol. 35, no. 22 , 1992 pages 4135 - 4142 A.H. NEWMAN ET AL. 'Synthesis and Evaluation of 3-Substituted 17-Methylmorhinan Analogs as Potential Anticonvulsant Agents'**
- **NIDA RES. MONOGR. (PROBL. DRUG DEPEND.) vol. 27 , 1979 pages 77 - 83 E. MOHACSI ET AL. 'A New Synthetic Codeine Substitute: (-)-3-Phenoxy-N-Methylmorphinan'**
- **METHODOL. SURV. BIOCHEM. ANAL. (DRUG METAB. ISOL. DETERM.) vol. 12 , 1983 pages 225 - 230 J.A.F. DE SILVA ET AL. 'Determination of 3-phenoxy-N-methylmorphinan and metabolites in plasma'**
- **BIOCHEM. PHARMACOL. vol. 31, no. 4 , 1982 pages 553 - 559 F.-J. LEINWEBER ET AL. 'Pharmacodynamic studies with (-)-3-phenoxy-N-methylmorphinan in rats'**
- **J. CHROMATOGR. vol. 232, no. 1 , 1982 pages 63 - 77 J.A.F. DE SILVA ET AL. 'Determination of the analgesic 3-phenoxy-N-methylmorphinan in plasma by gas chromatography using either positive chemical ionization mass spectrometric or nitrogen-phosphorus-specific detection analysis'**

Remarks:
The file contains technical information submitted after the application was filed and not included in this specification

EP 0 612 730 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.14.8/3.4

## Description

The present invention relates to the use of compounds of the formula

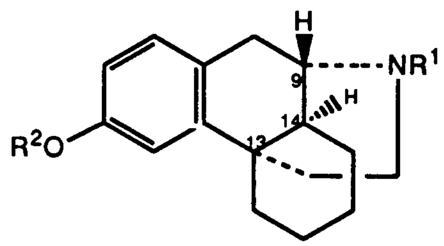

I

wherein $R^2$ is aryl, heteroaryl or a group of the formula $R^{20}$:

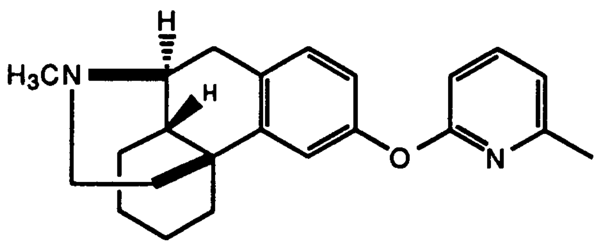

$R^{20}$

and $R^1$ is hydrogen, alkyl, a group of the formula $-C(Y,Y^1)CH_2OH$ or $-CH_2W$, wherein one of Y and $Y^1$ is hydrogen and the other is alkyl or both Y and $Y^1$ are alkyl and W is cycloalkyl, aryl, alkyl or allyl, and pharmaceutically acceptable salts thereof, for the manufacture of a medicament for reducing adverse effects of toxic injury to central neurons, particularly wherein the injury to central neurons is associated with ischemia, hypoxia, hypoglycemia, epilepsy, Huntington's disease or Alzheimer's disease, or for treating convulsions.

In another aspect, the invention relates to compounds of the formula:

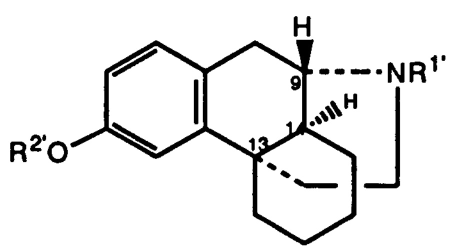

IA

wherein $R^{2'}$ is substituted or unsubstituted pyridyl, thiazolyl, thienyl, phenyl, or a group of the formula $R^{20}$, and $R^{1'}$is hydrogen, alkyl, or a group of the formula $-C(Y,Y^1)CH_2OH$, wherein one of $Y^1$ and Y is hydrogen and the other is alkyl or both Yand $Y^1$ are alkyl, provided that when $R^{2'}$ is pyridyl or phenyl, $R^{1'}$ is other than alkyl.

The compounds of formula I, as described above, reduce adverse effects of neurotoxic injury and thus are useful in the treatment of convulsions and neuro-degenerative diseases, such as, stroke, ischemia, hypoxia, hypoglycemia, epilepsy, Huntington's disease, Alzheimer's disease, cerebral palsy, pulmonary surgery or cardiac arrest, perinatal asphyxia, Olivopontocerebellar atrophy, anoxia, such as, from drowning, spinal cord injury and poisoning by exogenous N-methyl-D-aspartate (NMDA) poisons, such as, some forms of lathyrism. The compounds of formule I are non-competitive NMDA receptor antagonists. Accordingly, they are particularly useful as agents in the treatment of convulsions, neurodegenerative disease states including neurological disorders, such as epilepsy, stroke or cerebral ischemia.

Compounds of the invention are the non-opioid type of dextrorotatory morphinans having a ring system with the absolute stereochemistry of 9S, 14S, 13S, as illustrated in formula I.

As used herein, the term "alkyl", alone or in combination, denotes a straight- or branched-chain alkyl group containing 1 to 5 carbon atoms, preferably 1 to 4 carbon atoms, for example, methyl, ethyl, propyl, isopropyl, butyl and the like. The term "aryl", alone or in combination, denotes a group derived from an aromatic hydrocarbon such as, for example, phenyl or naphthyl, which may be unsubstituted or substituted by one or more substituents selected from alkyl, alkoxy, amino, nitro, halogen and hydroxy, preferably, alkyl or halogen. The term "heteroaryl" denotes an aryl group as

defined above having 5 or 6 members in the ring structure in which one or more of the ring carbon atoms is replaced with a hetero atom selected from the group consisting of N, S and O, which may be unsubstituted or substituted by one or more substituents selected from the group consisting of alkyl, nitro, amino, halogen, alkoxy and hydroxy. Suitable examples of heteroaryl include pyridyl, thienyl, furyl, thiazolyl, pyrimidyl, pyrrole, quinolyl and the like. The term "halogen" denotes chlorine, fluorine, iodine and bromine. The term "alkoxy", alone or in combination, denotes an alkyl group as defined earlier which is attached via an oxygen atom, examples of alkoxy groups are methoxy, ethoxy, propoxy, isopropoxy, butoxy, tert. butoxy and the like.

The invention also relates to pharmaceutical composition comprising a compound of formula IA or a pharmaceutically acceptable salt thereof, as well as to pharmaceutical compositions for reducing adverse affects of neurotoxic injury comprising a compound of formula I or pharmaceutically acceptable salt thereof.

In compounds of formula I, $R^2$ is preferably heteroaryl, particularly preferred is pyridyl or thiazolyl and $R^1$ is preferably alkyl or hydrogen, particularly preferred is methyl.

Compounds of formula I preferably used in the method of the invention include:

(+)-3-Phenoxy-N-methylmorphinan;
(+)-3-Thiazolyloxymorphinan;
(+)-3-[6-Methyl-2-(pyridinyl)oxy]morphinan;
(+)-17-Methyl-3-[(3-nitro-2-pyridinyl)oxy]morphinan;
(+)-β,β-Dimethyl-3-(2-pyridinyloxy)morphinan-17-ethanol;
(9β, 13β, 14β)-3-(2-Thienyloxy)-17-methylmorphinan;
(+)-17-Methyl-3-(3-pyridinyloxy)morphinan;
(9β, 13β, 14β)-2-[(17-Methylmorphinan-3-yl)oxy]-3-pyridinamine; and
(+)-3-(2-Pyrimidyloxy)-17-methylmorphinan; and
pharmaceutically acceptable salts thereof, particularly from the group consisting of:
(9β, 13β, 14β)-3-[(6-Bromo-2-pyridinyl)oxy]-17-methylmorphinan;
(9β, 13β, 14β)-17-Methyl-3-(2-thiazolyloxy)morphinan;
(+)-3-Phenoxymorphinan;
(+)-17-Methyl-3-[6-methyl-2-(pyridinyl)oxy]morphinan;
(+)-3-(2-Pyridyloxy)morphinan;
(+)-3-[(3-Nitro-2-pyridinyl)oxy]morphinan; and
pharmaceutically acceptable salts thereof, especially wherein the compound of formula I is (+)-3-(2-pyridyloxy)-N-methylmorphinan.

Particularly preferred compounds used in the method of the invention are:

(9β,13β,14β)-3-[(6-Bromo-2-pyridinyl)oxy]-17-methylmorphinan;
(9β,13β,14β)-17-Methyl-3-(2-thiazolyloxy)morphinan;
(+)-3-Phenoxymorphinan;
(+)-17-Methyl-3-[6-methyl-2-(pyridinyl)oxy]morphinan;
(+)-3-(2-Pyridyloxy)morphinan;
(+)-3-[(3-Nitro-2-pyridinyl)oxy]morphinan; especially (+)-3-(2-Pyridyloxy)-N-methylmorphinan; and pharmaceutically acceptable salts thereof.

A preferred group of the compounds of formula IA are those wherein $R^{2'}$ is substituted or unsubstituted pyridyl, thiazoyl or phenyl, especially preferred is pyridyl and $R^{1'}$ is hydrogen or alkyl, especially preferred is alkyl. Exemplary compounds of formula IA are:

(9β, 13β, 14β)-3-(2-Thiazolyloxy)morphinan;
(+)-β,β-Dimethyl-3-(2-pyridinyloxy)morphinan-17-ethanol;
(+)-17-Methyl-3-(3-pyridinyloxy)morphinan;
(9β, 13β, 14β)-2-[(17-Methylmorphinan-3-yl)oxy]-3-pyridinamine;
(+)-17-Methyl-3-(3-nitro-2-pyridinyl)morphinan; and pharmaceutically acceptable salts thereof. Preferred compounds of formula IA are:
(9β, 13β, 14β)-17-Methyl-3-(2-thiazolyloxy)morphinan;
(+)-3-(2-Pyridyloxy)morphinan;
(+)-17-Methyl-3-[6-methyl-2-(pyridinyl)oxy]morphinan;
(+)-3-[6-Methyl-2-(pyridinyl)oxy]morphinan;
(9β, 13β, 14β)-3-[(6-Bromo-2-pyridinyl)oxy]-17-methylmorphinan;
(+)-3-(3-Nitro-2-pyridinyl)morphinan;

(+)-3-Phenoxymorphinan, and pharmaceutically acceptable salts thereof.

The compounds of formula I can be prepared as hereinafter described in Schemes 1-4.

## SCHEME I

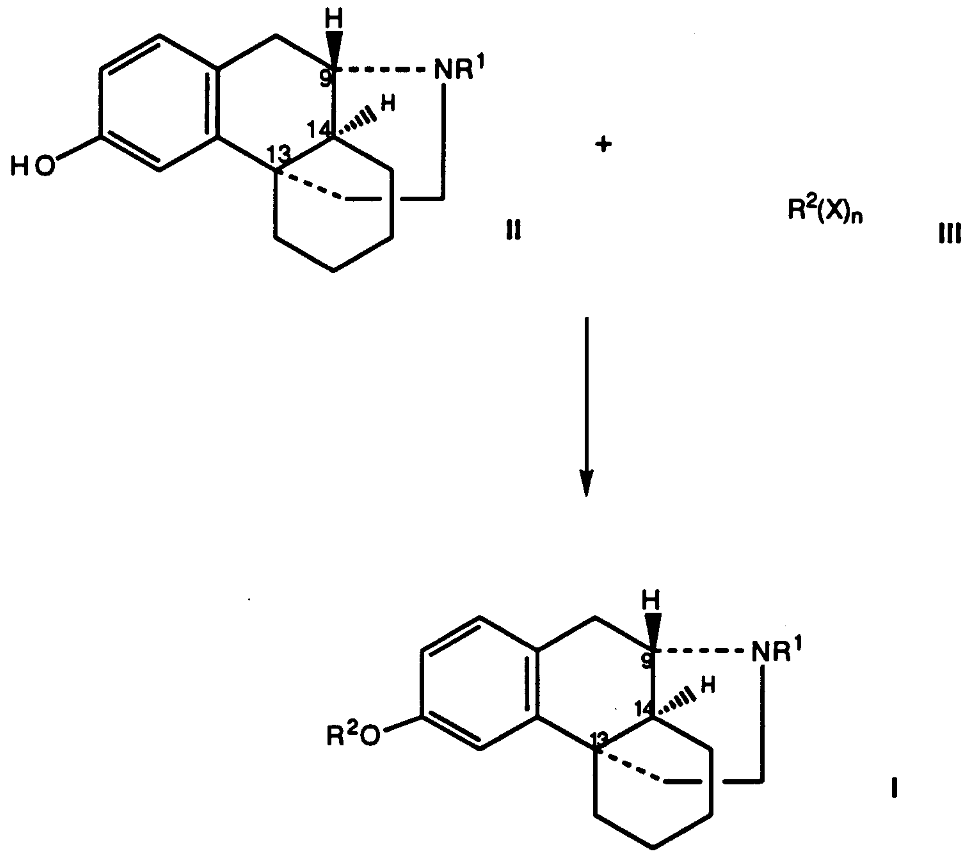

II + $R^2(X)_n$ III

I

wherein $R^2$ is as described above, $R^1$ is hydrogen, alkyl or a group of the formula $-CH_2W$, wherein W is as above, X is halogen, n is 1 or 2, provided that n is 2 when $R^2$ is a group of the formula $R^{20}$, further provided that $R^2$ is other than 3-nitro-2-pyridinyl, when $R^1$ is hydrogen.

The compounds of formulas II and III which are known compounds or can be prepared by known methods,.are reacted using the Ullmann reaction (Ann. 350, 1906, 83), to form the compound of formula I utilizing a copper catalyst. This reaction is carried out in an organic solvent in the presence of an inorganic alkali metal base. Any conventional organic solvent, preferably nitrobenzene, collidine, diglime or tertiary amines, can be utilized. Among the tertiary amines are included the cyclic tertiary amines such as pyridine and the tri-lower alkyl amines such as trimethyl amine, triethylamine, and the like. This reaction is also carried out in the presence of an inorganic base, such as an alkali metal base. Preferred bases are the alkali metal hydroxides such as potassium and sodium hydroxide as well as the alkali metal carbonates and bicarbonates such as sodium carbonate, potassium carbonate, sodium bicarbonate and potassium bicarbonate. The preferred inorganic base is a weak base such as potassium carbonate. Temperature and pressure are not critical: the reaction can be carried out at room temperature and atmospheric pressure. However, elevated temperatures can be utilized. Generally, it is preferred to utilize temperatures of from 100°-250°C. Examples of copper catalysts are cupric chloride, cupric bromide, cupric sulfate, cuprous iodide, a mixture of copper-bronze and metalic copper, with granual copper being preferred.

Compounds of the formula I wherein $R^2$ is pyridinyl, pyrimidyl or quinolinyl and $R^1$ is methyl, can be prepared as described in German Pat. No. 2.030981, and the compound of formula I wherein $R^2$ is phenyl and $R^1$ is methyl, as described in J. Med. Chem. 27, 1984, 1219.

## SCHEME 2

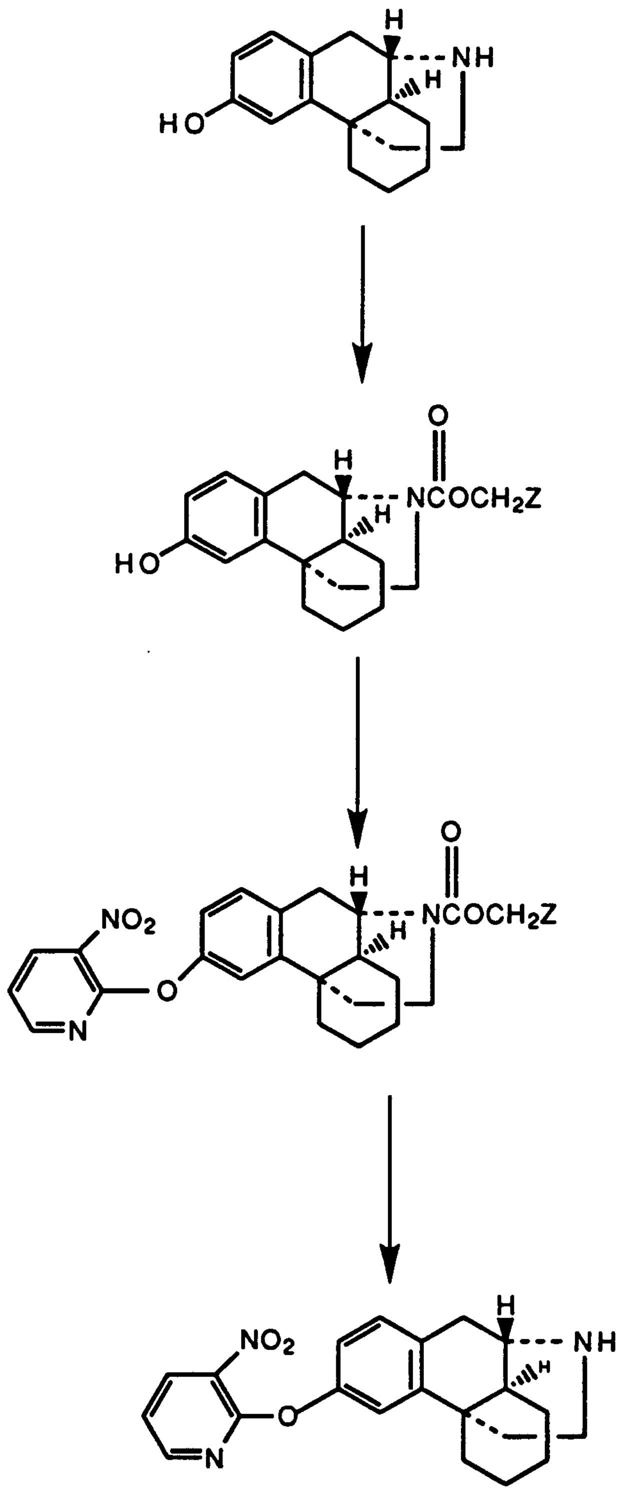

IIA

V

VI

IB

wherein Z is phenyl or methyl.

In accordance with scheme 2, the compound of formula IIA is converted to the compound of formula V with benzyl chloroformate or ethyl chloroformate. In carrying out this reaction, any inert organic solvent can be utilized, preferably aromatic hydrocarbon solvents, for example, benzene, toluene, methylene chloride, chloroform, and the like. Generally, this reaction is carried out in the presence of a base, preferably alkali metal carbonates such as sodium or potassium carbonate, or hydroxides such as sodium or potassium hydroxide. It is preferred to carry out this reaction at ice-bath

temperature. The compound of formula V is converted to the compound of formula VI with 2-chloro-3-nitropyridine using the Ullmann reaction described before. The compound VI is hydrolized to the compound IB by treating with an inorganic acid such as hydrochloric, sulfuric and the like. Among the preferred solvents are benzene and toluene. This reaction can be carried out at from 30-100° C and atmospheric pressure, preferably at room temperature.

The compound of formula IC, wich can be prepared as set forth in Scheme I,

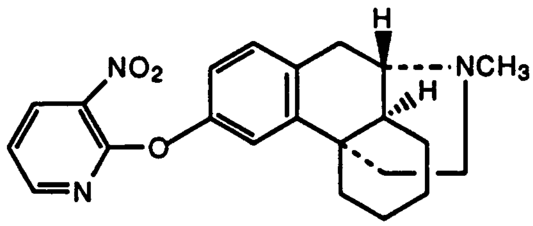

IC

can be reduced to the compound of formula ID

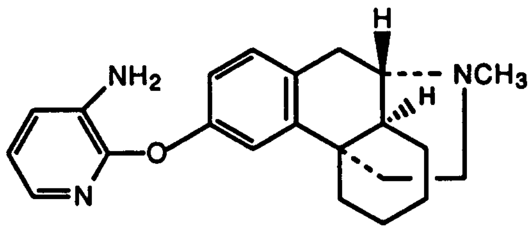

ID

using iron as reducing agent in an organic solvent such as ethanol or methanol, in the presence of an inorganic acid such as hydrochloric acid, at from 30° to 100° C and atmospheric pressure.

## SCHEME 3

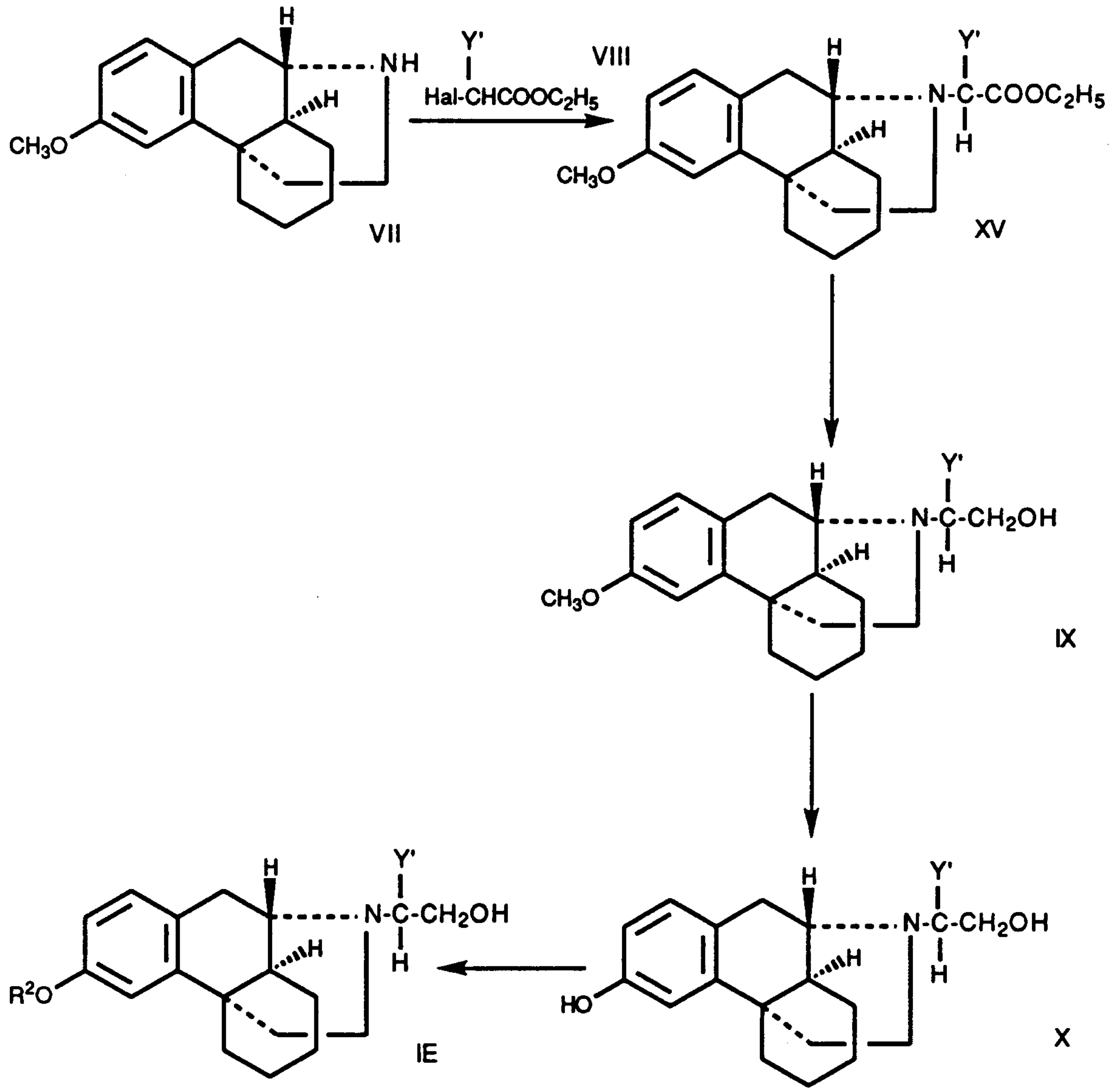


VII XV IX X IE

wherein Hal is halogen, Y' is alkyl and $R^2$ is as described above.

As provided in Scheme 3, the compound of formula VII, a known compound, is converted to the compound of formula XV by reacting with a compound of formula VIII, a known compound or which can be prepared by known methods. In carrying out this reaction, any inert organic solvent can be utilized as solvent. Generally, this reaction is carried out in DMF and in the presence of a weak base, e.g. an alkali metal carbonate or bicarbonate, such as sodium or potassium bicarbonate. Generally, it is preferred to carry out this reaction at about 100°C. The compound XV can be reduced to the compound IX by treatment with an alkali metal aluminum hydride, e.g. lithium aluminum hydride or a di(lower alkyl)aluminum hydride such as diisobutyl aluminum hydride. The compound IX can be converted to the compound X by ether cleavage, e.g. by treating the compound IX with pyridine hydrochloride or aqueous hydrogen bromide. The compound X can be converted to the compound IE by the Ulmann reaction described before.

## SCHEME 4

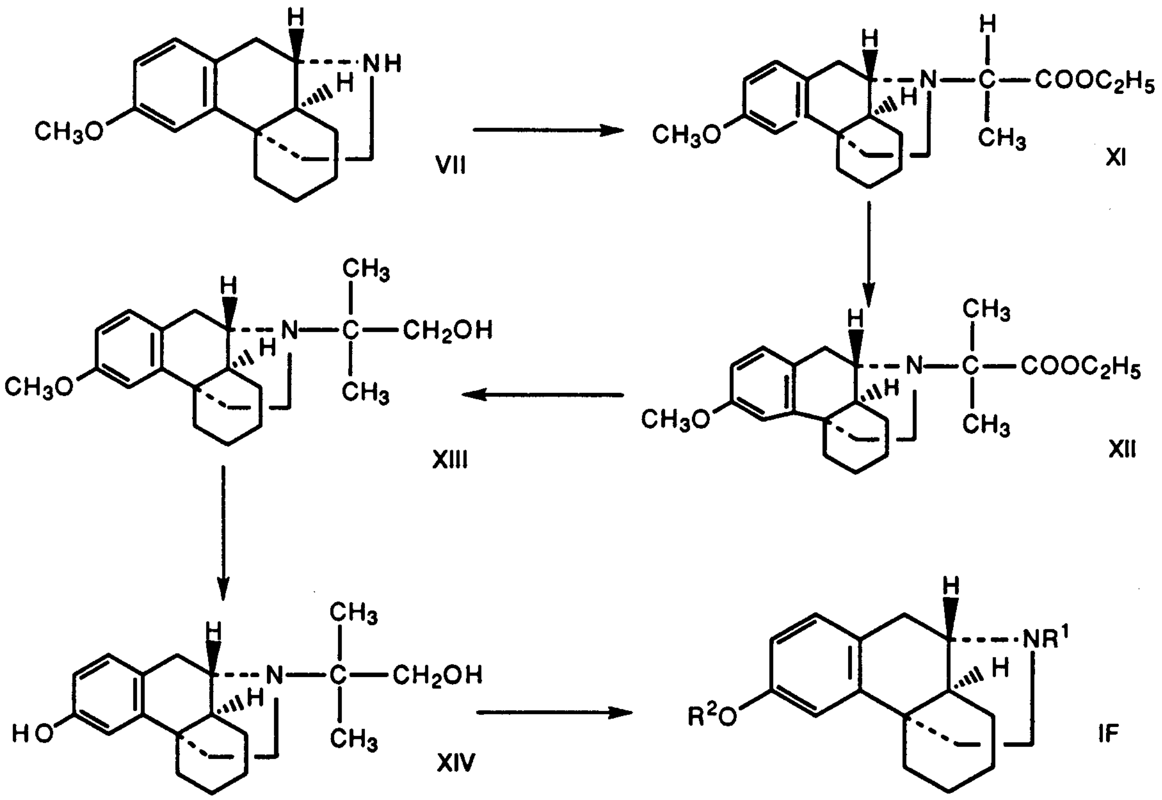

VII XI XII XIII XIV IF

wherein $R^2$ is aryl or heteroaryl and $R^1$ is a group of the formula $-C(CH_3)_2CH_2OH$. The compounds of formula IF, wherein $R^1$ is a group of the formula $-C(Y^1,Y)CH_2OH$, and Y and $Y^1$ are alkyl other than methyl or Y is H and $Y^1$ is alkyl can be prepared in a similar manner.

As set forth in Scheme 4, the compound of formula VII is converted to the compound of formula XI with ethyl 2-bromoproprionate. In carrying out this reaction, any inert organic solvent can be utilized as solvent. Generally, this reaction is carried out in DMF and in the presence of a weak base, preferably an alkali metal carbonate or bicarbonate, such as sodium or potassium bicarbonate. Generally, it is preferred to carry out this reaction at about 100°C. The compound XI is alkylated to the compound XII with methyl iodide. Carrying out this reaction, any inert organic solvent can be utilized as solvent, preferably THF, ethyl ether or dioxane and the reaction is carried out in the presence of a base, preferably lithium diisopropylamide, lithium N-cyclohexyl-N-isopropylamide or lithium diethylamide. Generally, it is preferred to carry out this reaction from -70° to -40°C.The compound XII is reduced to the compound XIII by treatment with an alkali metal aluminum hydride, such as, lithium aluminum hydride or a di(lower alkyl) aluminum hydride such as diisobutyl aluminum hydride. The compound XIII is converted to the compound XIV by ether cleavage, preferably with pyridine hydrochloride or aqueous hydrogen bromide. The compound of formula XIV is converted to the compound of formula IF by the Ullmann reaction conditions as described before.

As indicated above, the compounds of formula I are active as non-competitive NMDA receptor antagonists and are therefore, useful as neuroprotecting agents, for example, in the treatment of injury to central neurons associated with ischemia, hypoxia, hypoglycemia, epilepsy, Huntington's disease or Alzheimer's disease. This activity can be demonstrated by the following tests:

1. NMDA-Induced convulsions in mice

Male mice 45-54 days old and weighing 18-30 g are food-deprived for 24 hrs and then assigned in groups of 10 to various treatment conditions. In general, the method of Lehmann et al. described in J. Pharmacol. Exp. Ther. 240, 1987, 737-746, is used. Mice are administered a test compound intraperitoneally and are injected with NMDA (175 mg/kg i.p.) 30 minutes later. If any test compound is administered orally, the NMDA is injected 60 minutes later. NMDA is dissolved in 0.9% saline. Test compounds are likewise dissolved in 0.9% saline or, when necessary, suspended in 5% gum acacia. All injections are made in a volume of 0.2 ml/20 g body weight. The mice are observed for 30 minutes after NMDA

injection and four endpoints are noted: 1) time to the first clonic convulsions; 2) total number of mice exhibiting clonic convulsions; 3) total number of mice exhibiting tonic convulsions; and, 4) total number of mice that die. Results are represented as the number of mice protected at each dose level of a test compound; this gives an indication of the degree of protection at each dose and indicates whether there was a dose level at which complete protection occurred. A formula is used which adjusts for the number of mice "protected" in the control group, since the dose of NMDA used induces convulsions in less than 100 percent of the mice in the control group. The formula used is:
percent protection = 100 (E-C)/(10-C) , where E is the number of mice protected at the dose of the test compound, C is the number of mice not convulsing in the vehicle control group, and 10 is the number of mice per group. A given dose level of a compound is regarded as being active if this percent protection score is equal to or greater than 50 percent. The results are set forth in Table 1.

## Table 1

### NMDA-induced Anticonvulsant Test

| Salt | $R^2$ | $R^1$ | % mice protected |
|---|---|---|---|
| Oxalate | 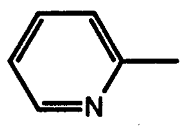 | $-CH_3$ | 86 |
| Fumarate | 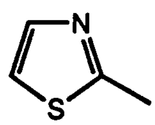 | $-CH_3$ | 86 |
| Hydrochloride | 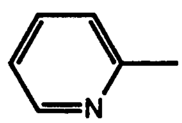 | 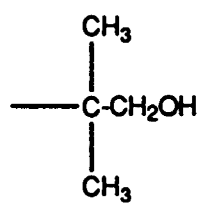 | 50 |
| Fumarate | 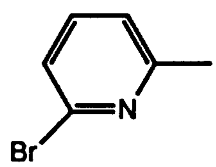 | $-CH_3$ | 67 |

2. Acute glutamate neurotoxicity

Single cell suspensions were prepared from embryonic rat cortices by digestion with dispase (2.4 U/ml) and subsequent trituration with fire polished Pasteur pipettes. The cells were then plated on poly-D-lysine coated microtiter plates (96 well/plate, $10^5$ cells/well) in a total volume of 100 µl essential medium supplemented with 10% horse serum and penicillin/streptomycin . Five days later, non-neuronal cell division was halted by exposure to $10^{-5}$ cytosine arabinoside combined with a 50% exchange of culture medium. The cultures were used for neurotoxicity assays from 8-12 days in vitro. Acute glutamate toxicity was performed as described in J. Koh et al. in Neurosci. 8, 1988, 185-196, in 100 ml of a control salt solution [CSS: 120 mM NaCl, 5.4 mM KCl, 0.8 mm $MgCl_2$, 1.8 mM $CaCl_2$, 25 mM Tris HCl (pH 7.4 at 25°C) and 15 mM glycose] with 500 mM glutamate for 5 to 30 min at room temperature with or without addition of substances to be tested.After washing, the cultures were maintained in 100 µl CSS overnight at 37°C. For quantitation of neurodegeneration, lactate dehydrogenase was measured in the cell culture supernatant as described by J.G. Klingman et al. in Neurosci. Meth. 31, 1990, 47-51. Percentage of neuronal degeneration was calculated taking the difference of unprotected and maximally protected cultures (with a reference NMDA-receptor antagonist) as 100%. From

dose response curves, $IC_{50}$ values were calculated. The results are set forth in Table 2.

**Table 2**

| Salt | $R^2$ | $R^1$ | $IC_{50}$ [µM] |
|---|---|---|---|
| Oxalate | 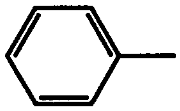 | $-CH_3$ | 6 |
| HCl | 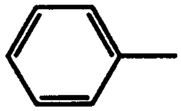 | -H | 1 |
| HCl | 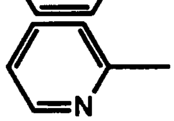 | $-CH_3$ | 4 |
| Maleate | 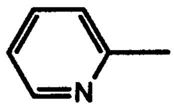 | -H | 6 |
| HCl | 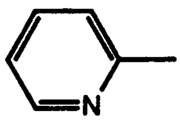 | 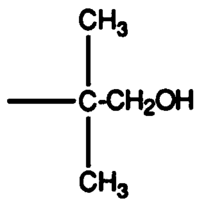 | 1 4 |

| | | | |
|---|---|---|---|
| Fumarate | 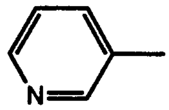 | $-CH_3$ | 6.4 |
| Fumarate | 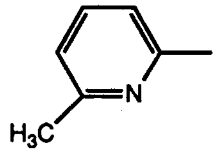 | $-CH_3$ | 0.2 |
| Fumarate | 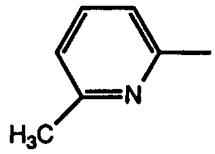 | -H | 1-2 |
| Fumarate | 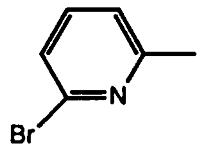 | $-CH_3$ | 1-2 |
| 2 HCl | 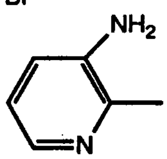 | $-CH_3$ | 14 |
| Fumarate | 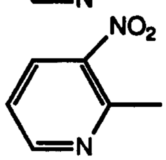 | $-CH_3$ | 5 |
| HCl | 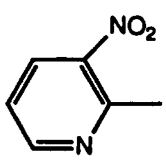 | -H | 0.5 |
| Fumarate | 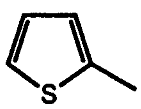 | $-CH_3$ | 13 |
| Fumarate | 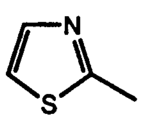 | $-CH_3$ | 5 |
| Maleate | 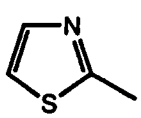 | -H | 8 |
| Fumarate | 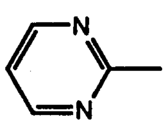 | $-CH_3$ | 4 |

The compounds of formula I form pharmaceutically acceptable acid addition salts with inorganic acids, such as hydrochloric acid, hydrobromic acid, sulfuric acid and phosphoric acid; and with organic acids, such as tartaric acid, oxalic acid, citric acid, camphorsulfonic acid, ethanesulfonic acid, toluenesulfonic acid, salicylic acid, ascorbic acid, maleic acid, succinic acid, formic acid, acetic acid and the like.

The compounds of formula I and their salts, as herein described, can be incorporated into standard pharmaceutical dosage forms, for example, for oral or parenteral application with the usual pharmaceutical adjuvant material, for example, organic or inorganic inert carrier materials, such as, water, gelatin, lactose, starch, magnesium stearate, talc, vegetable oils, gums, polyalkyleneglycols and the like. The pharmaceutical preparations can be employed in a solid form, for example, as tablets, suppositories, capsules, or in liquid form, for example, as solutions, suspensions or emulsions. Pharmaceutical adjuvant materials can be added and include preservatives, stabilizers, wetting or emulsifying agents, salts to change the osmotic pressure or to act as buffers. The pharmaceutical preparations can also contain other therapeutically active substances.

The daily dose of compounds of formula I to be administered varies with the particular compound employed, the chosen route of administration and the recipient. Representative of a method for administering the compounds of formula I is by the oral type administration route. By this route, oral formulation of a compound of formula I is preferably administered at a dose in the range of from 0.01 microgram to 0.15 microgram per day per kilogram.

The invention is further illustrated in the following examples.

## EXAMPLE 1

A solution of 5.1 g of (+)-3-hydroxy-N-methylmorphinan in 120 ml of pyridine was refluxed under nitrogen with 6.2 ml of bromobenzene, 6.9 g of potassium carbonate and 6.5 g of copper for four days. The mixture was filtered and the filtrate was concentrated under reduced pressure. The residue was partitioned between ether and 2N sodium hydroxide. The ether solution was washed with water, then dried and removal of the solvent gave 4.3 of (+)-3-phenoxy-N-methylmorphinan. A sample was recrystallized from hexane, mp 89-91°, $[\alpha]^{25}_D$ + 60.39° (c 0.92, methanol).

To the above base (4.19 g in acetone), a solution of 1.0 g of oxalic acid in acetone was added. The crude oxalate was recrystallized from isopropanol-acetone to give 2.5 g of (+)-3-phenoxy-N-methylmorphinan oxalate, mp 179-180°, $[\alpha]^{25}_D$ + 35.7° (c 1.00, methanol).

The fumarate salt was prepared in water from (+)-3-phenoxy-N-methylmorphinan and fumaric acid. The product was separated by filtration and lyophilized to give the amorphous fumarate as the semihydrate, mp 75-77°, $[\alpha]^{25}_D$ + 33.53° (c 1.02, methanol).

## EXAMPLE 2

A solution of 3.35 g of (+)-3-hydroxy-N-methylmorphinan benzene adduct (crystallized from benzene) in 60 ml of pyridine was heated at reflux until the temperature reached 114°. The reaction was then cooled and 20 ml of pyridine, 1.66 g of 2-bromopyridine, 2.0 g of potassium carbonate and 0.26 g of copper were added. The mixture was heated at reflux for 17 hours. It was cooled to room temperature, filtered and the filtrate was concentrated under reduced pressure. The residue was partitioned between ether and 10% sodium hydroxide. The ether solution was washed with water, then dried and removal of the solvent gave a product, which after crystallization from pet. ether afforded 2.4 g of (+)-3-(2-pyridyloxy)-N-methyl-morphinan, mp 114-116°, $[\alpha]25_D$ + 53.58° (c 1.10, methanol).

To the above base, 2.4 g in acetone, a solution of 1.4 g of oxalic acid in acetone was added. The crude oxalate was recrystallized from isopropanol-acetone to afford 2.9 g of (+)-3-(2-pyridyloxy)-N-methylmorphinan oxalate, mp 188-190°, $[\alpha]^{25}_D$ + 21.2° (c 1.00, methanol).

The fumarate salt was prepared and recrystallized from acetone, mp 84-86°, $[\alpha]^{25}_D$ + 16.67° (c 0.696, methanol).

The base, (6.0 g) in a mixture of 10 ml of methyl ethyl ketone and 2.8 ml of isopropanol was acidified with concentrated hydrochloric acid, then diluted with 15 ml of methyl ethyl ketone and allowed to crystallize for 48 hours. The salt was separated by filtration to give 1.5 g of (+)-3-(2-pyridyloxy)-17-methylmorphinan hydrochloride as the semihydrate, mp 116-120° , $[\alpha]^{25}_D$ + 23.11° (c 1.09, methanol).

## EXAMPLE 3

A solution of 3.35 g of (+)-3-hydroxy-N-methylmorphinan benzene adduct (crystallized from benzene) in 60 ml of pyridine was heated at reflux until the temperature reached 114°. The reaction was cooled, 10 ml of pyridine, 2.0 g of potassium carbonate, 1.1 g of 2-chloro-6-methylpyridine and 0.26 g of copper were added. The mixture was heated at reflux for 48 hours. It was cooled to room temperature, filtered and the filtrate was concentrated under reduced pressure. The residue was partitioned between ethyl acetate and 2N potassium hydroxide. The ethyl acetate solution was washed with brine, dried and the solvent was removed under reduced pressure. The product was dissolved in chloroform and chromatographed on silica gel, eluting with chloroform-methanol (95:5). Fractions 10-14 were combined and the solvent was removed to give 0.6 g of (+)-17-methyl-3-[6-methyl-2-(pyridinyl)oxy]morphinan, bp 170-175° (0.05 mm), $[\alpha]^{25}_D$ + 66.34° (c 1.00, methanol).

1.6 g of the base in acetone was treated with a solution of 0.8 g of fumaric acid in acetone. The fumarate was recrystallized from acetone to afford 1.0 g of (+)-17-methyl-3-[6-methyl-2-(pyridinyl)oxy]morphinan (E)-2-butene-dioate

hydrate, mp 128-130°, $[\alpha]^{25}_D$ + 38.08° (c 1.00, methanol).

**EXAMPLE 4**

A solution of 3.35 g of (+)-3-hydroxy-N-methylmorphinan benzene adduct (crystallized from benzene) in 60 ml of pyridine was heated at reflux until the temperature reached 114°. To the solution, 2.0 g of potassium carbonate, 1.8 g of 2-chloro-3-nitropyridine, and 0.3 g of copper were added. The mixture was heated at reflux for 30 hours then cooled to room temperature and filtered. The filtrate was concentrated at reduced pressure and the residue was partitioned between water and ethyl acetate. The ethyl acetate solution was washed with 2N sodium hydroxide then with brine and dried. Removal of the solvent gave after crystallization from ethyl acetate, 2.0 g of (+)-17-methyl-3-[(3-nitro-2-pyridinyl)oxy]morphinan, mp 188-189°, $[\alpha]^{25}_D$ + 51.38° (c 1.01, methanol).

To 1.7 g of the base in acetone, 0.553 g of fumaric acid was added and the crystals were separated by filtration to give 1.8 g of (+)-17- methyl-3-[(3-nitro-2-pyridinyl)oxy]morphinan (E)-2-butenedioate (2:3) salt, mp 107-108°, $[\alpha]^{25}_D$ + 24.33° (c 1.01, methanol).

**EXAMPLE 5**

A solution of 3.35 g of (+)-3-hydroxy-N-methylmorphinan benzene adduct (crystallized from benzene) in 60 ml of pyridine was heated at reflux until the temperature reached 114°. To the solution, 1.66 g of 3-bromo-pyridine, 2.07 g of anhydrous potassium carbonate and 0.257 g copper were added. The mixture was heated at reflux for 24 hours then cooled to room temperature and filtered. The filtrate was concentrated and the residue was extracted with ethyl acetate. The organic solution was washed with 2N potassium hydroxide then with brine and dried. Removal of the solvent gave after crystallization from acetone 1.4 g of (+)-17-methyl-3-(3-pyridinyloxy)morphinan, mp 125-126°, $[\alpha]^{25}_D$ + 64.40° (c 0.504, methanol).

0.668 g of the base was combined with 0.348 g of fumaric acid in 50 ml of water and heated until solution occurred. The crystals were collected by filtration and freeze dried to give 1.0 g of (+)-17-methyl-3-(3-pyridinyloxy)morphinan (E)-2-butenedioate (2:3) salt (4:3) molar hydrate, mp 68-70°, $[\alpha]^{25}_D$ + 27.12° (c 1.006, methanol).

**EXAMPLE 6**

A mixture of 3.35 g of (+)-3-hydroxy-N-methylmorphinan, 2.07 g of potassium carbonate, 1.2 g of 2-chloropyrimidine, 0.25 g of copper in 60 ml of pyridine was heated at reflux for 8 days then cooled to room temperature and filtered. The filtrate was concentrated and the residue was extracted with ethyl acetate. The ethyl acetate solution was washed with 1N sodium hydroxide, then with brine and dried. Removal of the solvent gave 2.0 g of (+)-3-(2-pyrimidyloxy)-17-methylmorphinan. A sample was recrystallized from ethyl acetate, mp 169-171°, $[\alpha]^{25}_D$ + 55.12° (c 0.923, methanol).

1.0 g of the base in acetone was combined with fumaric acid (0.4 g) and the crystals were separated by filtration to give 0.9 g of (+)-3-(2-pyrimidyloxy)-17-methylmorphinan (E)-2-butenedioate (2:3) salt, mp 115-117°, $[\alpha]^{25}_D$ + 20.56° (c 1.14, methanol).

**EXAMPLE 7**

A mixture of 6.7 g (+)-3-hydroxy-N-methylmorphinan, 3.6 g of 2-chloro-5-nitropyridine, 0.6 g of copper, 4.0 g of anhydrous potassium carbonate (powdered) in 120 ml of dry pyridine was stirred at reflux for 30 hours. It was cooled to room temperature then filtered and the filtrate was concentrated under reduced pressure. The residue was extracted with ethyl acetate. The organic solution was washed with 2N potassium hydroxide, then with brine and dried. The solvent was removed under reduced pressure and the residue was extracted with ether. Removal of the solvent gave 4.7 g of (9β, 13β, 14β)-17-methyl-3-[(5-nitro-2-pyridinyl)oxy]morphinan, mp 144-145°, $[\alpha]^{25}_D$ + 56.22° (c 1.03, methanol).

1.0 g of the base in acetone was combined with 0.45 g of fumaric acid and the crystals were separated by filtration to give 1.2 g of (9β, 13β, 14β)-17-methyl-3-[(5-nitro-2-pyridinyl)oxy]morphinan (E)-2-butenedioate (2:3) salt, mp 144-145°, $[\alpha]^{25}_D$ + 22.01° (c 1.00, methanol).

**EXAMPLE 8**

A mixture of 2.6 g of (+)-3-hydroxy-N-methylmorphinan, 2.0 g of powdered potassium carbonate, 0.3 g copper, 2.3 g of 2-bromo-thiazole in 60 ml of pyridine was heated at reflux for 24 hours then cooled to room temperature and filtered. The filtrate was concentrated under reduced pressure and the residue was extracted with ethyl acetate. The ethyl acetate solution was washed with 2N potassium hydroxide then brine, and the solvent was removed under reduced pressure. 2.2 g of the residue was chromatographed on silica gel, eluting with a mixture of chloroform: methanol: water: acetic acid (90:15:10:6, v/v). Fractions 6-18 were collected and the solvent was removed to give 1.4 g of (9β, 13β, 14β)-17-methyl-3-(2-thiazolyloxy)-morphinan. A sample was recrystallized from ether-pet. ether, mp 116-117°, $[\alpha]^{25}_D$ + 67.38° (c 1.01, methanol).

To 1.3 g of the base in ethanol, 0.45g of fumaric acid was added and the crystals were separated by filtration. The salt was recrystallized from acetone to give 0.7 g of (9β, 13β, 14β)-17-methyl-3-(2-thiazolyloxy)-morphinan (E)-2-butenedioate, mp 127-128°, $[\alpha]^{25}_D$ + 35.85° (c 1.03, methanol).

**EXAMPLE 9**

A solution of 3.35 g of (+)-3-hydroxy-N-methylmorphinan benzene adduct (recrystallized from benzene) in 50 ml of dry pyridine was heated at reflux until the temperature reached 114°. After cooling, 2.0 g of powdered potassium carbonate, 0.8 g of 2-bromothiophene and 0.26 g of copper were added to the solution and heated at reflux for 20 hours. After cooling, the mixture was filtered and the filtrate was concentrated under reduced pressure. The product was chromatographed on silica gel, eluting with a mixture of chloroform: methanol: water: acetic acid (90:15:10:6, v/v). Fractions 5-9, after removal of the solvents, gave 0.7 g of (9β, 13β, 14β)-3-(2-thienyloxy)-17-methylmorphinan, bp 205-210° (0.25 mm), $[\alpha]^{25}_D$ + 58.06° (c 0.261, methanol).

0.8 g of the base in acetone was treated with 0.24 g of fumaric acid and the crystals were separated to give 0.6 g of (9β, 13β, 14β)-3-(2-thienyloxy)-17-methylmorphinan (E)-2-butenedioate, mp 155-156°, $[\alpha]^{25}_D$ + 41.58° (c 1.02, methanol).

**EXAMPLE I0**

A solution of 3.35 g of (+)-3-hydroxy-N-methylmorphinan benzene adduct (crystallized from benzene) in 60 ml of dry pyridine was heated at reflux until the temperature reached 114°. To the cooled solution, 2.0 g of potassium carbonate, 1.8 g of 2-chloroquinoline and 0.3 g copper were added and heated at reflux for 30 hours. It was cooled, filtered and concentrated under reduced pressure. The residue was extracted with ethyl acetate. The organic solution was washed with 2N potassium hydroxide then with water and concentrated under reduced pressure. The residue was chromatographed on silica gel, eluting with a mixture of chloroform: methanol: water: acetic acid (90:15:10:6, v/v). Fractions 9-18, after removal of the solvent, gave after recrystallization from acetone, 0.9 g of (9β, 13β, 14β)-17-methyl-3-(2-quino-linyloxy)morphinan, mp 142-143°, $[\alpha]^{25}_D$ + 78.48° (c 0.81, methanol).

0.8 g of the base in acetone was treated with 0.5 g of fumaric acid and the crystals were separated to give 1.0 g of (9β, 13β, 14β)-17-methyl-3-(2-quinolinyloxy)morphinan (E)-2-butenedioate (2:3) salt, mp 161-162°, $[\alpha]^{25}_D$ + 32.97° (c 0.98, methanol).

**EXAMPLE II**

A solution of 3.35 g of (+)-3-hydroxy-N-methylmorphinan benzene adduct (crystallized from benzene) in 60 ml of dry pyridine was heated at reflux until the temperature reached 113°. To the cooled solution, 2.0 g of potassium carbonate, 0.3 g of copper and 2.8 g of 2,6-dibromopyridine were added. The mixture was heated at reflux for 17 hours, then cooled and filtered. The filtrate was concentrated under reduced pressure and the residue was extracted with ethyl acetate. The ethyl acetate solution was washed with 2N potassium hydroxide then with brine and the solvent was removed under reduced pressure. The residue was chromatographed on silica gel, eluting with a mixture of chloroform: methanol: water: acetic acid (90:15:10:6, v/v). Fractions 8-19, after removal of the solvents and recrystallization of the residue, gave 1.2 g of (9β, 13β, 14β)-3-[(6-bromo-2-pyridinyl)oxy]-17-methylmorphinan, mp 123-124°, $[\alpha]^{25}_D$ + 74.76° (c 1.03, methanol).

0.8 g of the base in acetone was treated with 0.5 g fumaric acid and the crystals were separated to give 1.1 g of (9β, 13β, 14β)-3-[(6-bromo-2-pyridinyl)oxy]-17-methylmorphinan (E)-2-butenedioate (2:3) salt, mp 152-153°, $[\alpha]^{25}_D$ + 46.12° (c 1.02, methanol).

**EXAMPLE 12**

A mixture of 2.6g of (+)-3-hydroxy-N-methylmorphinan, 2.0 g of 2-chloro-4-methyl-5-nitropyridine, 2.0 g of powdered potassium carbonate, 0.3 g of copper and 60 ml pyridine was heated at reflux for 48 hours. After cooling, the mixture was filtered and the filtrate was concentrated under reduced pressure. The residue was partitioned between ethyl acetate and brine. The organic solution was washed with 2N potassium hydroxide then with brine and dried. Removal of the solvent and recrystallization of the crude product from ether gave 2.0 g of (9β, 13β, 14β)-17-methyl-3-[(4-methyl-5-nitro-2-pyridinyl)oxy]-morphinan, mp 145-146°, $[\alpha]^{25}_D$ + 61.21° (c 1.04, methanol).

1.4 g of the base in acetone on treatment with 0.5 g of fumaric acid gave 1.7 g of (9β, 13β, 14β)-17-methyl-3-[(4-methyl-5-nitro-2-pyridinyl)oxy]-morphinan (E)-butenedioate, mp 198-200°, $[\alpha]^{25}_D$ + 21.90° (c 1.08, methanol).

**EXAMPLE 13**

A solution of 6.7 g of (+)-3-hydroxy-N-methylmorphinan benzene adduct (crystallized from benzene) in 120 ml pyridine was heated at reflux until the temperature reached 113°. To the cooled solution under nitrogen, 4.0 g of powdered potassium carbonate, 0.6 g of copper and 5.6 g of 2,6-dibromopyridine were added and heated at reflux for 20 hours. It was cooled, filtered and the filtrate was concentrated at reduced pressure. The residue was extracted with ethyl acetate and the organic solution was washed with 2N potassium hydroxide. The solvent was removed and the residue was chromotographed on silica gel, eluting with a mixture of chloroform: methanol: water: acetic acid (90:30:10:6, v/v). Fractions 34-40, after removal of solvents, gave 1.0 g of (9β, 13β, 14β)-2,6-pyridinylbis(oxy)-3,3'-bis(17-methyl-morphinan), bp 280-285° (0.25 mm), $[\alpha]^{25}_{D}$ + 83.50° (c 0.94, methanol).

1.0 g of the base in acetone, on treatment with hydrogen chloride, after recrystallization from ethanol--acetone, gave 0.7 g of (9β, 13β, 14β)-2,6-pyridinylbis(oxy)-3,3'-bis(17-methylmorphinan) dihydrochloride sesquihydrate, mp 257-258°, $[\alpha]^{25}_{D}$ + 72.68° (c 0.8, methanol).

**EXAMPLE 14**

A mixture of 12.9 g of (+)-3-methoxymorphinan, 10.0 g of ethyl-2-bromopropionate, 8.4 g of anhydrous sodium bicarbonate and 100 ml of DMF was heated at 100° for 15 hrs. The reaction mixture was concentrated to about 40 ml and partitioned between water and ether. The aqueous solution was extracted with ether and the combined solutions were washed with water and dried. Removal of the solvent under reduced pressure gave 17.1 g of (+)-3-methoxy-N-(2-ethoxycarbonyl-2-ethyl)morphinan. A sample was distilled, bp 180° (0.07 mm), $[\alpha]^{25}_{D}$ + 70.78° (c 0.97, methanol).

**EXAMPLE 15**

Under nitrogen, to a solution of 1.75 g of diisopropylamine in 25 ml of THF at -40° was added a hexane solution containing 10.9 ml of 1.6 M of n-butyllithium. The resulting solution was stirred at this temperature for 15 minutes then cooled to -70°. A solution of 5.6 g of (+)-3-methoxy-N-(2-ethoxycarbonyl-2-ethyl)morphinan in 20 ml of dry THF was added dropwise to the above lithium diisopropylamide. The resulting solution was allowed to warm-up to -40° and a solution of 2.5 g of methyl iodide in 10 ml of THF was added dropwise. The reaction mixture was stirred at room temperature for 16 hrs. The mixture was partitioned between water and ether. The ether solution was washed with water and dried. Removal of the solvent at reduced pressure gave 5.7 g of (+)-3-methoxy-N-(2-ethoxycarbonyl-2-propyl) morphinan. A sample was distilled, bp 180-185° (0.08 mm), $[\alpha]^{25}_{D}$ + 82.5° (c 1.00, methanol).

**EXAMPLE 16**

To a suspension of 1.2 g of $LiAlH_4$ in 100 ml of THF, was added dropwise a solution of 5.7 g of (+)-3-methoxy-N-(2-ethoxycarbonyl-2-propyl)morphinan in 50 ml of THF. After the mixture had been refluxed for 12 hrs under nitrogen, it was cooled to room temperature and 20 ml of ethyl acetate followed by 10 ml of water were added dropwise. The resulting suspension was filtered and the filtrate was dried. Removal of the solvent under reduced pressure afforded 4.8 g of (+)-3-methoxy-N-(2-hydroxymethyl-2-propyl)morphinan, mp 83-84°. A sample was distilled, bp 190° (0.08 mm), $[\alpha]^{25}_{D}$+74.5° (c 1.00, methanol).

A sample of 1.5 g of the base, was treated with hydrogen chloride in ethyl acetate. Recrystallization from ethanol-ethyl acetate gave 1.53 g of (+)-3-methoxy-N-(2-hydroxymethyl-2-propyl)morphinan hydrochloride , mp 215-216°, $[\alpha]^{25}_{D}$+33.94° (c_1.00, methanol).

**EXAMPLE 17**

A mixture of 1.2 g of (+)-3-methoxy-N-(2-hydroxymethyl-2-propyl)morphinan and 11 ml of 62% hydrobromic acid was heated at 60° under nitrogen for 5 hrs. The excess reagent was removed under reduced pressure and the residue was crystallized from methanol-ethanol to give 1.4 g of (+)-3-hydroxy-N-(2-hydroxymethyl-2-propyl)morphinan hydrobromide, mp 165-167°, $[\alpha]^{25}_{D}$+38.29° (c 1.01, methanol).

2.0 g of the base, prepared above from the hydrobromide, was treated with hydrogen chloride in acetone. After recrystallization from acetone, 1.9 g of (+)-3-hydroxy-β,β-dimethylmorphinan-17-ethanol hydrochloride, mp 254-255°, $[\alpha]^{25}_{D}$+41.75° (c 0.512, methanol) were obtained.

**EXAMPLE 18**

A solution of 2.9 g of (+)-3-hydroxy-N-(2-hydroxymethyl-2-propyl) morphinan in 60 ml of dry pyridine was heated at reflux until the temperature reached 114°. To the cooled solution was added 1.66 g of 2-bromopyridine, 0.3 g copper

and 1.4 g of powdered potassium carbonate then the mixture was heated at reflux for 20 hours. It was cooled to room temperature, filtered and the filtrate was concentrated at reduced pressure. The residue was partitioned between ether and 2N potassium hydroxide. The ether solution was washed with 2N potassium hydroxide then with brine and the solvent was removed under reduced pressure. The residue was chromotographed on silica gel, eluting with methylene chloride. Fractions 11-15, after removal of the solvent, gave 2.3 g of (+)-β,β-dimethyl-3-(2-pyridinyloxy)morphinan-17-ethanol as an amorphous substance, $[\alpha]^{25}_D$ + 61.09° (c 0.772, methanol).

2.3 g of the base in acetone gave, on treatment with hydrogen chloride, 1.5 g (54%) of (+)-β,β-dimethyl-3-(2-pyridinyloxy)-morphinan-17-ethanol dihydrochloride hydrate, mp. 105-107°, $[\alpha]^{25}_D$ + 27.33° (c 1.19, methanol).

**EXAMPLE 19**

A mixture of 1.9 g of (+)-17-methyl-3-[(3-nitro-2-pyridinyl)oxy] morphinan, 10 ml ethanol, 3 ml 6N hydrochloric acid and 3.0 g of iron was heated on the steam bath for 30 minutes, then 3 ml of 6N hydrochloric acid were added. The mixture was cooled to room temperature, filtered then the filtrate was concentrated and made basic with concentrated ammonium hydroxide. The aqueous suspension was extracted with ethyl acetate, the organic solution was washed with brine and dried. The solvent was removed and the product was chromatographed on silica gel, eluting with a mixture of chloroform: methanol: water: acetic acid (90:30:10:6; v/v). Fractions 3-14, after removal of the solvents, gave 1.3 g of (9β, 13β, 14β)-2-[(17-methylmorphinan-3-yl)oxy]-3-pyridineamine, bp 225-230° (0.25 mm), $[\alpha]^{25}_D$ + 41.35° (c 0.906, methanol).

1.3 g of the base in acetone, on treatment with hydrogen chloride gave 0.9g of (9β, 13β, 14β)-2-[(17-methylmorphinan-3-yl)oxy]-3-pyridineamine dihydrochloride, mp 260-261°, $[\alpha]^{25}_D$ + 19.83° (c 0.98, methanol).

**EXAMPLE 20**

A solution of 2.4 g of (+)-3-hydroxymorphinan in 60 ml of pyridine was heated at reflux until the temperature reached 114°. To the cooled solution was added 20 ml of pyridine, 1.66 g of 2-bromopyridine, 2.0 g of powdered potassium carbonate and 0.26 g of copper. The mixture was heated at reflux for 20 hours. It was cooled to room temperature, filtered and the filtrate was concentrated under reduced pressure. The residue was partitioned between ether and 2N potassium hydroxide. The ether solution was washed with brine, dried and the solvent was removed under reduced pressure to give 3.1 g of (+)-3-(2-pyridyloxy)morphinan. A sample was crystallized from ether-pet. ether, mp 87-88°, $[\alpha]^{25}_D$ + 36.17° (c 0.976, methanol).

3.4 g of the base in acetone was treated with maleic acid. The maleate was recrystallized from acetone to afford 3.0 g of (+)-3-(2-pyridyloxy)morphinan (Z)-butenedioate, mp 164-165°, $[\alpha]^{25}_D$ + 15.00° (c 0.833, methanol).

**EXAMPLE 21**

A mixture of 2.4 g of (+)-3-hydroxymorphinan, 1.7 g of bromo-benzene, 600 mg of copper and 1.5 g of powdered potassium carbonate in 60 ml of pyridine was heated at reflux under nitrogen for 5 days. It was cooled to room temperature, filtered and the filtrate was concentrated under reduced pressure. The residue was partitioned between ether and 2N potassium hydroxide. The ether solution was washed with benzene, dried and the solvent was removed under reduced pressure. The product was treated in ethanol-ethyl acetate with hydrogen chloride to give 2.4 g of (+)-3-phenoxy-morphinan hydrochloride. A sample was recrystallized from methanol, mp 320-321°, $[\alpha]^{25}_D$ + 15.00° (c 0.72, methanol).

A sample of the hydrochloride was converted to the base using dilute ammonium hydroxide as the base and ethyl acetate for extraction. A sample was crystallized from ether, mp 65-66°, $[\alpha]^{25}_D$ + 42.83° (c 0.68, methanol).

**EXAMPLE 22**

A mixture of 2.4 g of (+)-3-hydroxymorphinan, 1.4 g of 2-chloro-6-methylpyridine, 1.5 g of powdered potassium carbonate and 300 mg of copper in 60 ml of pyridine was heated at reflux for 3 days. It was cooled to room temperature, filtered and the filtrate was concentrated at reduced pressure. The residue was partitioned between ethyl ether and 2N potassium hydroxide. The ethyl ether solution was washed with brine, dried and the solvent was removed under reduced pressure. The product was chromatographed on silica gel, eluting with methylene chloride: methanol (90:30, v/v). Fractions 30-59 were combined and the solvent was removed to give 0.6 g of (+)-3-[6-methyl-(2-pyridinyl)oxy] morphinan, bp 195-197° (0.05 mm), $[\alpha]^{25}_D$ + 48.54° (c 1.13, methanol).

0.6g of the base was treated in ethanol with 600 mg of fumaric acid to give 0.6 g of (+)-3-[6-methyl-(2-pyridinyl)oxy]morphinan (E)-2-butenedioate. A sample was recrystallized from ethanol, , mp 240-242°, $[\alpha]^{25}_D$ + 36.43° (c 1.06, methanol).

**EXAMPLE 23**

A mixture of 2.4 g of (+)-3-hydroxymorphinan, 1.6 g of 2-bromothiazole, 1.4 g of powdered potassium carbonate and 0.6 g of copper in 60 ml of pyridine was refluxed with stirring under nitrogen for 24 hours. The mixture was filtered and the filtrate was concentrated under reduced pressure. The residue was partitioned between ethyl acetate and water. The organic phase was washed in 2N potassium hydroxide, then brine and dried. The solvent was removed gave and the product was chromato-graphed on silica gel, eluting with a mixture of chloroform: methanol: water: ethyl acetate [90:15:10:6 (v/v)]. Fractions 4-10, after removal of the solvents, yielded 0.6 g of (+)-3-thiazolyloxymorphinan as amorphous compound, $[\alpha]^{25}_D$ + 51.82° (c 0.55, methanol).

0.6 g of the base were treated in acetone with 0.2g of maleic acid. Recrystallization from acetone gave 0.50 g of (+)-3-thiazolyloxymorphinan maleate, mp 179-180°, $[\alpha]^{25}_D$ + 32.51° (c 0.99, methanol).

**EXAMPLE 24**

To a mixture of 2.4 g of (+)-3-hydroxymorphinan, 10 ml of methylene chloride and 10 ml of water was added simultaneously 2.0 g of benzyl chloroformate in 5 ml of methylene chloride and 4.4 ml of 10% sodium hydroxide at ice-bath temperature over 5 min. The mixture was stirred at room temperature for 1.5 hours and the organic solution was separated. The aqueous layer was extracted with methylene chloride, then the combined organic solutions were washed with brine and dried. Removal of the solvent gave 3.8 g of benzyl-3-hydroxy-morphinan-N-carboxylate as an amorphous substance, $[\alpha]^{25}_D$ + 138.6° (c 0.43, methanol).

**EXAMPLE 25**

A mixture of 10.80 g of benzyl-(+)-3-hydroxymorphinan-N- carboxylate, 4.6 g of 2-chloro-3-nitropyridine, 4.0g of powdered potassium carbonate and 0.8 g copper in 100 ml of pyridine was heated at reflux for 20 hours. The mixture was filtered and the filtrate was concentrated under reduced pressure. The residue was washed with heptane and then partitioned between ethyl acetate and 2N potassium hydroxide. The organic solution was washed with brine, dried and filtered. The filtrate was treated with norite, filtered and the solvent was removed under reduced pressure. The product was chromatographed on silica gel, eluting with methylene chloride. Fractions 5-32 yielded, after removal of the solvent, 4.5 g of benzyl-(+)-3-[(3-nitro-2-pyridinyl) oxy]morphinan-N-carboxylate as an amorphous compound, $[\alpha]^{25}_D$ + 123.9° (c 1.0, methanol).

**EXAMPLE 26**

A mixture of 0.5 g of benzyl-(+)-3-[(3-nitro-2-pyridinyl)oxy]-morphinan-N-carboxylate, 8 ml of concentrated hydrochloric acid in 8 ml of benzene was stirred at room temperature for 6 hours then poured onto ice water. The aqueous suspension was extracted with ether then chilled and made basic with concentrated ammonium hydroxide. The aqueous suspension was extracted with ethyl acetate. The combined ethyl acetate solutions were dried and removal of the solvent gave 0.3 g of (+)-3-[(3-nitro-2-pyridinyl)oxy]morphinan. A sample was recrystallized from ethyl acetate-ether, mp 161-162°, $[\alpha]^{25}_D$ + 60.62° (c 1.04, methanol).

0.3 g of the base was treated with hydrogen chloride. Recrystallization from 1N hydrochloric acid gave 0.3 g of (+)-3-[(3-nitro-2-pyridinyl)oxy]morphinan hydrochloride, as the hydrate, mp 149-150°, $[\alpha]^{25}_D$ + 25.80° (c 1.15, methanol).
The following galenical composition were prepared in a manner known per se.

**EXAMPLE A**

| Tablet formulation (wet granulation) | | | | |
|---|---|---|---|---|
| Ingredients | mg/tablet | | | |
| (+)-3-(2-Pyridyloxy)-N-methyl morphinan hydrochloride, (9β, 13β, 14β)-17-Methyl-3-(2-thiazolyloxy)morphinan (E)-2-butenedioate or (9β, 13β, 14β)-3[(6-Bromo-2-pyridinyl)oxy]-17-methylmorphinan (E)-2-butenedioate | 12.5 | 25 | 100 | 500 |
| Anhydrous lactose | 117.5 | 105 | 30 | 150 |
| Pregelatinized starch | 6.0 | 6 | 6 | 30 |
| Microcrystalline cellulose | 30.0 | 30 | 30 | 150 |
| Magnesium stearate | 1.0 | 1 | 1 | 5 |
| TOTAL: | 167.0 | 167 | 167 | 835 |

**EXAMPLE B**

| Capsule formulation | | | | |
|---|---|---|---|---|
| Item Ingredients | mg/capsule | | | |
| (+)-3-(2-Pyridyloxy)-N-methyl morphinan hydrochloride, (9β, 13β, 14β)-17-Methyl-3-(2-thiazolyloxy)morphinan (E)-2-butenedioate or (9β, 13β, 14β)-3[(6-Bromo-2-pyridinyl)oxy]-17-methylmorphinan (E)-2-butenedioate | 12.5 | 25 | 100 | 500 |
| Corn starch | 95.5 | 83 | 8 | 40 |
| Modified starch | 4.0 | 4 | 4 | 20 |
| Talc | 4.0 | 4 | 4 | 20 |
| Magnesium stearate | 1.0 | 1 | 1 | 5 |
| TOTAL: | 117.0 | 117 | 117 | 585 |

**Claims**

**1.** The use of a compound of the formula:

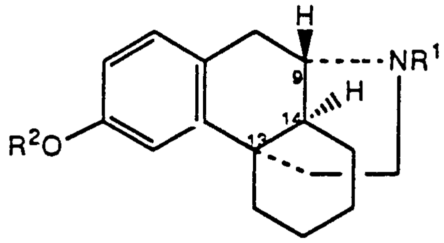

I

wherein $R^2$ is phenyl or naphthyl, which may be substituted by one or more substituents selected from ($C_1$-

$C_5$) alkyl, ($C_1$-$C_5$) alkoxy, amino, nitro, halogen and hydroxy, a 5 or 6 membered hydrocarbon, in which one or more of the ring carbon atoms is replaced with N, S and O, which may be substituted by the above or a group of the formula $R^{20}$

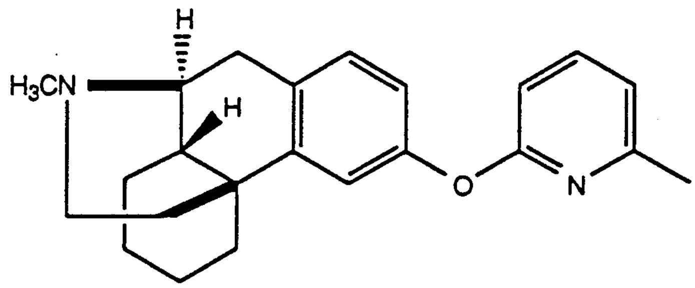

$R^{20}$

and $R^1$ is hydrogen, ($C_1$-$C_5$) alkyl, a group of the formula -C(Y,$Y^1$,)$CH_2OH$ or -$CH_2W$, wherein one of Y and $Y^1$ is hydrogen and the other is ($C_1$-$C_5$) alkyl or both Y and $Y^1$ are ($C_1$-$C_5$) alkyl and W is ($C_3$-$C_6$) cycloalkyl, phenyl or naphthyl which may be substituted by one or more substituents selected from ($C_1$-$C_5$) alkyl, ($C_1$-$C_5$) alkoxy, amino, nitro, halogen and hydroxy, or allyl,
and pharmaceutically acceptable salts thereof, for the manufacture of a medicament for reducing adverse effects of toxic injury to central neurons, particularly wherein the injury to central neurons is associated with ischemia, hypoxia, hypoglycemia, epilepsy, Huntington's disease or Alzheimer's disease, or for treating convulsions.

2. The use of Claim 1, wherein in the compound of formula I, $R^2$ is phenyl, napthyl pyridyl, thienyl, furyl, thiazoyl, quinolyl or pyrimidyl and $R^1$ is hydrogen or ($C_1$-$C_5$) alkyl.

3. The use of Claim 2, wherein the compound of formula I is selected from the group consisting of:

(+)-3-Phenoxy-N-methylmorphinan;
(+)-3-Thiazolyloxymorphinan;
(+)-3-[6-Methyl-2-(pyridinyl)oxy]morphinan;
(+)-17-Methyl-3-[(3-nitro-2-pyridinyl)oxy]morphinan;
(+)-β,β-Dimethyl-3-(2-pyridinyloxy)morphinan-17-ethanol;
(9β, 13β, 14β)-3-(2-Thienyloxy)-17-methylmorphinan;
(+)-17-Methyl-3-(3-pyridinyloxy)morphinan;
(9β, 13β, 14β)-2-[(17-Methylmorphinan-3-yl)oxy]-3-pyridinamine; and
(+)-3-(2-Pyrimidyloxy)-17-methylmorphinan; and
pharmaceutically acceptable salts thereof, particularly from the group consisting of:
(9β, 13β, 14β)-3-[(6-Bromo-2-pyridinyl)oxy]-17-methylmorphinan;
(9β, 13β, 14β)-17-Methyl-3-(2-thiazolyloxy)morphinan;
(+)-3-Phenoxymorphinan;
(+)-17-Methyl-3-[6-methyl-2-(pyridinyl)oxy]morphinan;
(+)-3-(2-Pyridyloxy)morphinan;
(+)-3-[(3-Nitro-2-pyridinyl)oxy]morphinan; and
pharmaceutically acceptable salts thereof, especially wherein the compound of formula I is (+)-3-(2-pyridyloxy)-N-methylmorphinan.

4. Compounds of the formula

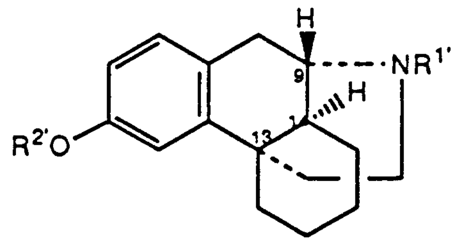

IA

wherein $R^{2'}$ is substituted or unsubstituted pyridyl, thiazolyl, thienyl, phenyl wherein the substituents are selected from ($C_1$-$C_5$) alkyl, ($C_1$-$C_5$) alkoxy, amino nitro, halogen and hydroxy, or a group of the formula $R^{20}$ as in claim 1, and $R^{1'}$ is hydrogen, ($C_1$-$C_5$) alkyl, or a group of the formula -C($Y^1$,$Y^2$)$CH_2$OH, wherein one of Y and $Y^1$ is hydrogen and the other is ($C_1$-$C_5$) alkyl or both Y and $Y^1$ are ($C_1$-$C_5$) alkyl, provided that when $R^{2'}$ is pyridyl or phenyl, $R^{1'}$ is other than ($C_1$-$C_5$) alkyl, particularly compounds of formula IA wherein $R^{2'}$ is pyridyl or phenyl, $R^{1'}$ is hydrogen, and pharmaceutically acceptable salts thereof.

**5.** The compounds of claim 4, selected from the group consisting of

(9β, 13β, 14β)-17-Methyl-3-(2-thiazolyloxy)morphinan;
(9β, 13β, 14β)-3-(2-Thiazolyloxy)morphinan;
(+)-3-(2-Pyridyloxy)morphinan;
(+)-17-Methyl-3-(3-pyridinyloxy)morphinan;
(+)-17-Methyl-3-[6-methyl-2-(pyridinyl)oxy]morphinan;
(+)-3-[6-Methyl-2-(pyridinyl)oxy]morphinan;
(9β, 13β, 14β)-3-[(6-Bromo-2-pyridinyl)oxy]-17-methylmorphinan;
(9β, 13β, 14β)-2-[(17-Methylmorphinan-3-yl)oxy]-3-pyridinamine;
(+)-17-Methyl-3-(3-nitro-2-pyridinyl)morphinan;
(+)-3-[3-Nitro-2-pyridinyl)morphinan;
(+)-3-Phenoxymorphinan; and
pharmaceutically acceptable salts thereof, particularly (+)-β,β-Dimethyl-3-(2-pyridinyl-oxy)morphinan-17-ethanol.

**6.** The compounds of claim 4 or 5, for reducing adverse effects of toxic injury to central neurons, particularly wherein the injury to central neurons is associated with ischemia, hypoxia, hypoglycemia, epilepsy, Huntington's disease or Alzheimer's disease, or for treating convulsions.

**7.** A process for the manufacture of an ether of formula I in claim 1, which process comprises

a) reacting a corresponding alcohol with a compound of formule $R^2(X)_n$, wherein $R^2$ is as in claim 1, X is halogen and n is 1 or 2, provided n is 2, when $R^2$ is a group of formula $R^{20}$ as defined in claim 1, further provided that $R^2$ is other than 3-nitro-2-pyridinyl, when $R^1$ is H, or
b) reacting a compound of the formula

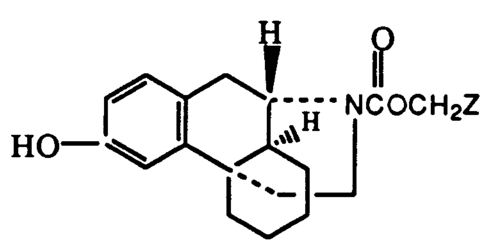

V

wherein Z is phenyl or methyl, to form a compound of the formula

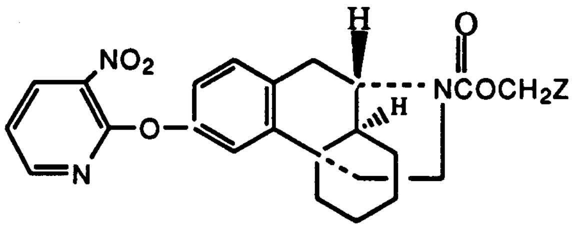

VI

wherein Z is phenyl or methyl, which is reacted to form a compound of the formula

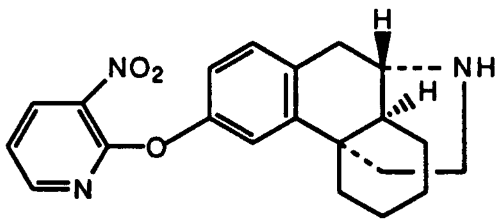

IB

8. Pharmaceutical composition particularly for reducing adverse effects of neurotoxic injury, comprising a compound as in claim 4 or 5.

9. Pharmaceutical composition for reducing adverse effects of neurotoxic injury comprising a compound of formula I as in claim 1 or a pharmaceutically acceptable salt thereof.

**Patentansprüche**

1. Verwendung einer Verbindung der Formel

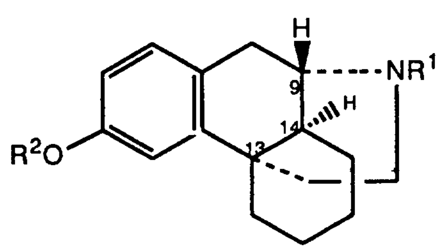

I

worin

$R^2$ Phenyl oder Naphthyl, die durch einen oder mehrere Substituenten substituiert sein können und die ausgewählt sind aus der Gruppe ($C_1$-$C_5$)-Alkyl, ($C_1$-$C_5$)-Alkoxy, Amino, Nitro, Halogen und Hydroxy; oder ein 5 oder 6 gliedriger Kohlenwasser stoffring, worin ein oder mehrere Ring-Kohlenstoffatome durch N, S oder O ersetzt sind und wie oben beschrieben substituiert sein können; oder eine Gruppe der Formel $R^{20}$

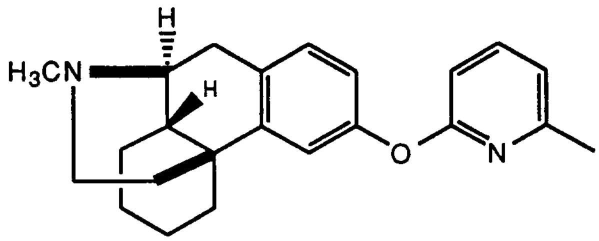

R20

bedeutet,

und

$R^1$ Wasserstoff, ($C_1$-$C_5$)-Alkyl, eine Gruppe der Formel -C(Y,$Y^1$)$CH_2OH$ oder -$CH_2W$ bedeutet, worin eines von Y und $Y^1$ Wasserstoff und das andere ($C_1$-$C_5$)-Alkyl oder Y und $Y^1$ beide ($C_1$-$C_5$)-Alkyl bedeuten und W ($C_3$-$C_6$)-Cycloalkyl, Allyl, Phenyl oder Naphthyl bedeutet, wobei Phenyl und Naphthyl durch einen oder mehrere Substituenten substituiert sein können, die ausgewählt sind aus der Gruppe ($C_1$-$C_5$)-Alkyl, ($C_1$-$C_5$)-Alkoxy, Amino, Nitro, Halogen und Hydroxy,

sowie deren pharmazeutisch annehmbare Salze für die Herstellung eines Medikamentes zur Verringerung nachteiliger Effekte von neurotoxischen Schäden zentraler Nervenzellen, insbesondere wenn die Schädigung zentraler Nervenzellen mit Ischämie, Hypoxie, Hypoglykämie, Epilepsy, der Huntington'schen oder Alzheimer'schen Krankheit in Zusammenhang steht, oder zur Behandlung von Krämpfen.

**2.** Verwendung gemäss Anspruch 1, worin $R^2$ in Formel I Phenyl, Naphthyl, Pyridyl, Thienyl, Furyl, Thiazolyl, Quinolyl oder Pyrimidyl bedeutet und R1 Wasserstoff oder ($C_1$-$C_5$)-Alkyl ist.

**3.** Verwendung gemäss Anspruch 2, worin die Verbindung der Formel I ausgewählt ist aus einer Gruppe, bestehend aus

(+)-3-Phenoxy-N-Methylmorphinan;
(+)-3-Thiazolyloxymorphinan;
(+)-3-[6-Methyl-2-([pyridinyl)oxy]morphinan;
(+)-17-Methyl-3-[(3-nitro-2-pyridinyl)oxy]morphinan;
(+)-β,β-Dimethyl-3-(2-pyridinyloxy)morphinan-17-ethanol;
(+)-17-Methyl-3-(3-pyridinyloxy)morphinan;
(9β,13β,14β)-2[(17-Methylmorphinan-3-yl)oxy]-3-pyridinamin; und
(+)-3-(2-Pyrimidyloxy)-17-methylmorphinan; und deren
pharmazentisch annehmbare Salze, insbesondere aus der Gruppe bestehend aus:
(9β,13β,14β)-3-[(6-Brom-2-pyridinyl)oxy]-17-methylmorphinan;
(9β,13β,14β)-17-Methyl-3-(2-thiazolyloxy)morphinan;
(+)-3-Phenoxymorphinan;
(+)-17-Methyl-3-[6-methyl-2(pyridinyl)oxy]morphinan;
(+)-3-(2-Pyridyloxy)morphinan;
(+)-3-[(3-Nitro-2-pyridinyl)oxy]morphinan; und deren pharmazeutisch annehmbare Salze, insbesondere worin die Verbindung der Formel I (+)-3-(2-Pyridyloxy)-N-methylmorphinan bedeutet.

**4.** Verbindungen der Formel

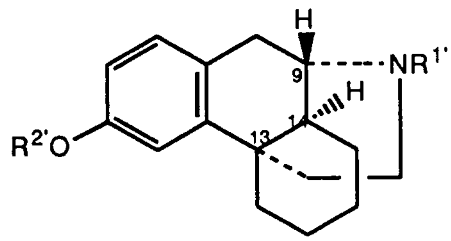

IA

worin $R^{2'}$ substituiertes oder unsubstituiertes Pyridyl, Thiazolyl, Thienyl oder Phenyl bedeutet und worin die Substituenten ausgewählt sind aus einer Gruppe, bestehend aus $(C_1\text{-}C_5)$-Alkyl, $(C_1\text{-}C_5)$-Alkoxy, Amino, Nitro, Halogen oder Hydroxy, oder eine Gruppe der Formel $R^{20}$, wie im Anspruch 1 beschrieben, bedeutet und $R^{1'}$ Wasserstoff oder $(C_1\text{-}C_5)$-Alkyl oder eine Gruppe der Formel $-C(Y\ Y^1)CH_2OH$ bedeutet und worin eines von Y und $Y^1$ Wasserstoff und das andere $(C_1\text{-}C_5)$-Alkyl oder beide $(C_1\text{-}C_5)$-Alkyl bedeuten, vorausgesetzt, dass, wenn $R^{2'}$ Pyridyl oder Phenyl bedeutet, $R^{1'}$ von $(C_1\text{-}C_5)$-Alkyl verschieden ist, insbesondere Verbindungen der Formel IA, worin $R^{2'}$ Pyridyl oder Phenyl bedeutet und $R^{1'}$ Wasserstoff ist, sowie deren pharmazeutisch annehmbare Salze.

**5.** Verbindungen gemäss Anspruch 4, ausgewählt aus einer Gruppe bestehend aus

(9β,13β,14β)-17-Methyl-3-(2-thiazolyloxy)morphinan;
(9β,13β,14β)-3-(2-Thiazolyloxy)morphinan;
(+)-3-(2-Pyridyloxy)morphinan;
(+)-17-Methyl-3-(3-pyridinyloxy)morphinan;
(+)-17-Methyl-3-[6-methyl-2-(pyridinyl)oxy]morphinan;
(+)-3-[6-Methyl-2-(pyridinyl)oxy]morphinan;
(9β,13β,14β)-3-[(6-Brom-2-pyridinyl)oxy]-17-methylmorphinan;
(9β,13β,14β)-2-[(17-Methylmorphinan-3-yl)oxy]-3-pyridinamin;
(+)-17-Methyl-3-(3-nitro-2-pyridinyl)morphinan;
(+)-3-[3-Nitro-2-pyridinyl)morphinan;
(+)-3-Phenoxymorphinan; und pharmazeutisch annehmbare Salze davon, insbesondere (+)-β,β-Dimethyl-3-(2-pyridinyl-oxy) morphinan-17-ethanol.

**6.** Verbindungen gemäss Ansprüchen 4 oder 5 zur Verringerung nachteiliger Effekte von neurotoxischen Schäden zentraler Nervenzellen, insbesondere, wenn die Schädigung zentraler Nervenzellen mit Ischämie Hypoxie, Hypoglykämie, Epilepsy, der Huntington'schen oder Alzheimer' schen Krankheit in Zusammenhang steht, oder zur Behandlung von Krämpfen.

**7.** Verfahren zur Herstellung eines Ethers der Formel I gemäss Anpsruch 1, wobei das Verfahren beinhaltet:

a) Reaktion eines entsprechenden Alkohols mit einer Verbindung der Formel $R^2(X)_n$, worin $R^2$ die Bedeutung wie im Anspruch 1 besitzt, X ist Halogen und n ist 1 oder 2, vorausgesetzt, dass n=2 ist, wenn $R^2$ eine Gruppe der Formel $R^{20}$, die so wie im Anspruch 1 definiert ist, bedeutet, und weiterhin vorausgesetzt, dass $R^2$ von 3-Nitro-2-pyridinyl verschieden ist, wenn $R^1$ Wasserstoff ist, oder

b) Reaktion einer Verbindung der Formel

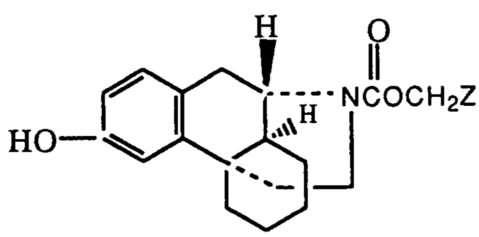

V

worin Z Phenyl oder Methyl ist, zu einer Verbindung der Formel

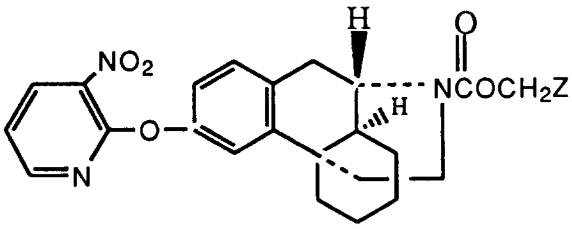

VI

worin Z Phenyl oder Methyl ist, die weiterhin zu einer Verbindung der Formel

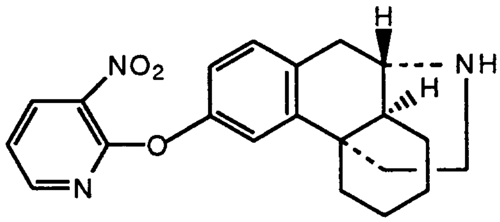

IB

umgesetzt wird.

8. Pharmazeutische Zusammensetzung zur Verringerung nachteiliger Effekte von neurotoxischen Schäden, enthaltend eine Verbindung gemäss Ansprüchen 4 oder 5.

9. Pharmazeutische Zusammensetzung zur Verringerung nachteiliger Effekte von neurotoxischen Schäden, enthaltend eine Verbindung der Formel I gemäss Anspruch 1 oder deren pharmazeutisch annehmbares Salz.

## Revendications

1. Utilisation d'un composé de formule :

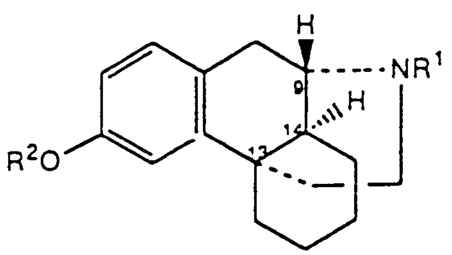

I

dans laquelle R2 est un groupe phényle ou naphtyle, qui peut être substitué par un ou plusieurs substituants choisis parmi un groupe alkyle en $C_1$ à $C_5$, alcoxy en $C_1$ à $C_5$, amino, nitro, halogène et hydroxy, ou un hydrocarbure à 5 ou 6 chaînons, dans lequel un ou plusieurs atomes de carbone du cycle sont remplacés par N, S et O, qui peut être substitué par les groupes précédants, ou un groupe de formule $R^{20}$

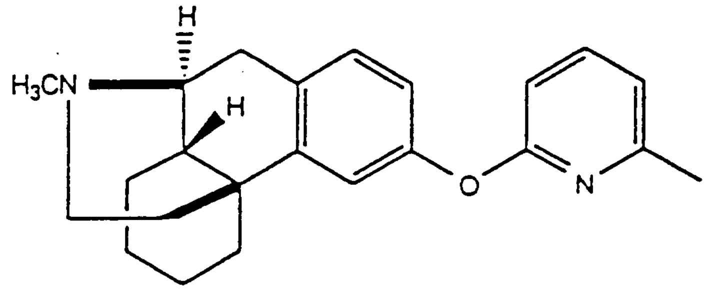

et R1 est un atome d'hydrogène, un groupe alkyle en $C_1$ à $C_5$, un groupe de formule -C(Y, $Y^1$)$CH_2OH$ ou -$CH_2W$, dans lequel l'un des groupes Y et $Y^1$ est un atome d'hydrogène et l'autre est un groupe alkyle en $C_1$ à $C_5$, ou les groupes Y et $Y^1$ sont tous deux un groupe alkyle en $C_1$ à $C_5$ et W est un groupe cycloalkyle en $C_3$ à $C_6$, phényle ou naphtyle, qui peut être substitué par un ou plusieurs substituants choisis parmi un alkyle en $C_1$-$C_5$, alcoxy en $C_1$-$C_5$, amino, nitro, halogène et hydroxy, ou allyle,

et des sels pharmaceutiquement acceptables de celui-ci, pour l'obtention d'un médicament destiné à réduire les effets secondaires de lésions d'origine toxique de neurones centraux, en particulier dans laquelle la lésion de neurones centraux est associé à l' ischémie, l'hypoxie, l'hypoglycémie, l'épilepsie, la maladie de Huntington ou la maladie d'Alzheimer, ou au traitement des convulsions.

2. Utilisation selon la revendication 1, dans laquelle dans le composé de formule I, R2 est un groupe phényle, naphtyle, pyridyle, thiényle, furyle, thiazole, quinolyle ou pyrimidyle et $R^1$ est un atome d'hydrogène ou un groupe alkyle en $C_1$ à $C_5$.

3. Utilisation selon la revendication 2, dans laquelle le composé de formule I est choisi dans le groupe consisté de :

(+)-3-phénoxy-N-méthylmorphinane;
(+)-3-thiazolyloxymorphinane;
(+)-3-[6-méthyl-2-(pyridinyl)oxy]morphinane;
(+)-17-méthyl-3-[3-(nitro-2-pyridinyl)oxy]morphinane;
(+)-β,β -diméthyl-3-(2-pyridinyloxy)morphinan-17-éthanol;
(9β, 13β, 14β)-3-(2-thiényloxy)-17-méthylmorphinane;
(+)-17-méthyl-3-(3-pyridinyloxy)morphinane;
(9β, 13β, 14β)-2-[(17-méthylmorphinan-3-yl)oxy]-3-pyridinamine; et
(+)-3-(2-pyrimidyloxy)-17-méthylmorphinane; et
des sels pharmaceutiquement acceptables de ceux-ci, en particulier dans le groupe consisté de :
(9β, 13β, 14β)-3-[6-(bromo-2-pyridinyl)oxy]-17-méthylmorphinane;
(9β, 13β, 14β)-17-méthyl-3-(2-thiazolyloxy)morphinane;
(+)-3-phénoxymorphinane;
(+)-17-méthyl-3-[6-méthyl-2-(pyridinyl)oxy]morphinane;
(+)-3-(2-pyridyloxy)morphinane;
(+)-3-[(3-nitro-2-pyridinyl)oxy]morphinane; et
des sels pharmaceutiquement acceptables de ceux-ci, en particulier dans laquelle le composé de formule I est le (+)-3-(2-pyridyloxy)-N-méthylmorphinane.

4. Composés de formule

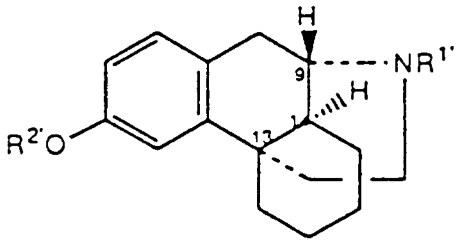

IA

dans laquelle R2' est un groupe pyridyle, thiazolyle, thiényle, phényle substitué ou non substitué dans lequel les substituants sont choisis parmi alkyle en $C_1$ à $C_5$, alcoxy en $C_1$ à $C_5$, amino, nitro, halogène et hydroxy, ou un groupe de formule $R^{20}$ selon la revendication 1, et $R^{1'}$ est un atome d'hydrogène, un groupe alkyle en $C_1$ à $C_5$, ou un groupe de formule $-C(Y^1, Y^2)CH_2OH$, dans lequel l'un des groupes Y et $Y^1$ est un atome d'hydrogène et l'autre est un groupe alkyle en $C_1$ à $C_5$ ou les groupes Y et $Y^1$ sont tous deux des groupes alkyle en $C_1$ à $C_5$, à la condition que, lorsque $R^{2'}$ est un groupe pyridyle ou phényle, $R^{1'}$ soit autre qu'un groupe alkyle en $C_1$ à $C_5$, en particulier des composés de formule IA dans laquelle R2' est un groupe pyridyle ou phényle, $R^{1'}$ est un atome d'hydrogène, et les sels pharmaceutiquement acceptables de ceux-ci.

5. Composés selon la revendication 4, choisis dans le groupe consistant de

(9β, 13β, 14β)-17-méthyl-3-(2-thiazolyloxy)morphinane;
(9β, 13β, 14β)-3-(2-thiazolyloxy)morphinane;
(+)-3-(2-pyridyloxy)morphinane;
(+)-17-méthyl-3-(3-pyridinyloxy)morphinane;
(+)-17-méthyl-3-[6-méthyl-2-(pyridinyl)oxy]morphinane;
(+)-3-[6-méthyl-2-(pyridinyl)oxy]morphinane;
(9β, 13β, 14β)-3-[(6-bromo-2-pyridinyl)oxy]-17-méthylmorphinane;
(9β, 13β, 14β)-2-[(17-méthylmorphinan-3-yl)oxy]3-pyridinamine;
(+)-17-méthyl-3(3-nitro-2-pyridinyl)morphinane;
(+)-3-((3-nitro-2-pyridinyl)morphinane;
(+)-3-phénoxymorphinane; et
des sels pharmaceutiquement acceptables de ceux-ci, en particulier le (+)-β, β -diméthyl-3-(2-pyridinyloxy)morphinan-17-éthanol.

6. Composés selon la revendication 4 ou 5, pour réduire les effets secondaires de lésions toxiques des neurones centraux, en particulier dans lesquels la lésion des neurones centraux est associée à l'ischémie, l'hypoxie, l'hypoglycémie, l'épilepsie, la maladie de Huntington ou la maladie d'Alzheimer, ou pour traiter les convulsions.

7. Procédé d'obtention d'un éther de formule I selon la revendication 1, lequel procédé comprend

a) la mise en réaction d'un alcanol avec un composé de formule $R^2(X)_n$, dans lequel $R^2$ est tel que défini à la revendication 1, X est un groupe halogène et n est égal à 1 ou 2, à la condition que n soit égal à 2, lorsque $R^2$ est un groupe de formule $R^{20}$ tel que défini à la revendication 1, à la condition en outre que $R^2$ soit autre qu'un groupe 3-nitro-2-pyridinyle, lorsque $R^1$ est H, ou
b) la mise en réaction d'un composé de formule

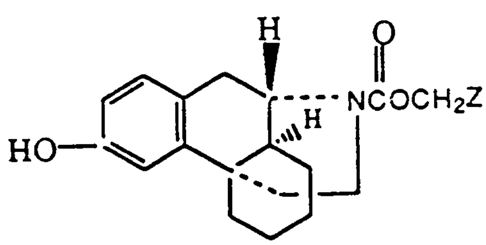

V

dans laquelle Z est un groupe phényle ou méthyle, en vue de former un composé de formule

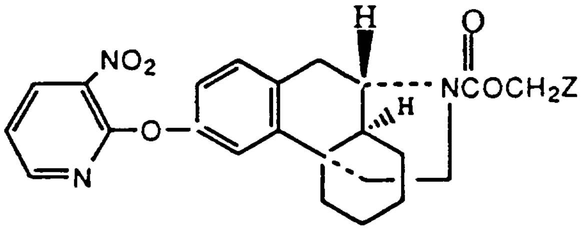

VI

dans laquelle Z est un groupe phényle ou méthyle, qui est mis à réagir pour donner un composé de formule

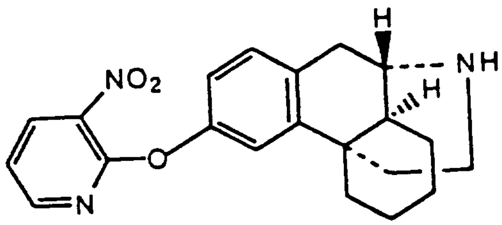

IB

8. Composition pharmaceutique destinée en particulier à réduire les effets secondaires de lésions neurotoxiques, comprenant un composé selon la revendication 4 ou 5.

9. Composition pharmaceutique pour réduire les effets secondaires de lésions neurotoxiques comprenant un composé de formule I selon la revendication 1 ou un sel pharmaceutiquement acceptable de celui-ci.